# EUROPÄISCHE PATENTSCHRIFT

(11) **EP 0 362 741 B1**
(45) Veröffentlichungstag und Bekanntmachung des Hinweises auf die Patenterteilung: **01.02.1995**
(21) Anmeldenummer: 89118170.3
(22) Anmeldetag: 30.09.1989
(51) Int. Cl.: A61L 2/18, A61C 19/00

(54) **Desinfektionsgerät**
Disinfecting apparatus
Appareil de désinfection

(30) Priorität: 05.10.1988 DE 8812539 U
(43) Veröffentlichungstag der Anmeldung: 11.04.1990
(73) Patentinhaber: Dürr-Dental GmbH & Co. KG, D-74321 Bietigheim-Bissingen (DE)
(72) Erfinder: Hofmann, Hans-Joachim, Dipl.-Ing., D-7064 Remshalden 3 (DE)
(74) Vertreter: Ostertag, Reinhard

(56) Entgegenhaltungen:
- DE-A- 3 706 826
- FR-A- 2 255 082

## Beschreibung

Die Erfindung betrifft ein Gerät zum Desinfizieren von dentalen Objekten wie Gebißabdrücken.

Aufgrund des zunehmenden Bewußtseins der Infektionsgefahren in Zahnarztpraxen und dentalen Labors ist es wünschenswert, alle Objekte, die mit Speichel oder Blut eines Patienten in Berührung gekommen sind, zu desinfizieren. Eine durchgängige Desinfektion derartiger Objekte wird aber in der Praxis nur dann lückenlos durchgeführt, wenn das Desinfizieren mit nur geringem Aufwand erledigt werden kann und schon von der apparativen Seite her gewährleistet ist, daß die Desinfektion auch in ausreichendem Ausmaße durchgeführt wird. Beide Gesichtspunkte sind bei einer Desinfektion unter Verwendung von Sprays oder Tauchbädern nicht gegeben.

Aus der DE-A-37 06 826 ist hierzu ein Desinfektionsgerät bekannt, welches einen dichten Kasten aufweist, in welchen von einer Desinfektionsmittel-Sprühdüse flüssiges Desinfektionsmittel und Wasser wahlweise abgegeben werden können. Die Steuerung des Wasserstromes und der Desinfektionsmittelabgabe erfolgt unter Verwendung angesteuerter Ventile, die in zum Frischwassernetz bzw. zu einer Desinfektionsmittel-Vorratsflasche führende Leitungen eingefügt sind.

Dies hat den Nachteil, daß die abgegebene Desinfektionsmittelmenge je nach der Dauer der Betätigung des in die Desinfektionsmittel-Speiseleitung geschalteten Ventiles schwanken kann, wodurch die Qualität oder die Wirtschaftlichkeit der Desinfektion in Frage gestellt werden kann.

Durch die vorliegende Erfindung soll daher ein Gerät zum Desinfizieren von dentalen Objekten geschaffen werden, bei welchem die Desinfektion mit vorgegebener Intensität Zwangsläufig erfolgt und die Handhabung so einfach ist, daß das Gerät vom Benutzer im Alltag angenommen wird.

Diese Aufgabe ist erfindungsgemäß gelöst durch ein Desinfektionsgerät gemäß Anspruch 1.

Bei dem erfindungsgemäßen Desinfektionsgerät kann man im Inneren des spritzwasserdichten Kastens einen intensiven Nebel an Desinfektionsmittel erzeugen, ohne daß eine Beeinträchtigung des Raumes gegeben ist, in welchem sich der Benutzer befindet. Darüber hinaus ist gewährleistet, daß für jeden Desinfektionsvorgang ein genau vorgegebenes Volumen an Desinfektionsmittel verwendet wird. Dieses Desinfektionsmittelvolumen kann aus der Vorratsflasche für eine große Anzahl aufeinander folgender Desinfektionszyklen entnommen werden. Man hat so insgesamt eine einfache und sichere Handhabung.

Bei einem erfindungsgemäßen Desinfektionsgerät erhält man eine sehr genaue Dosierung des Desinfektionsmittels bei sehr einfachem Aufbau der Dosiereinrichtung. Die Meßbohrung kann in der Praxis einfach am in der Regel aus Kunststoff gespritzten oder geschäumten Gehäuse angeformt werden. Das mit der Meßbohrung zusammenarbeitende Verschlußstück ist ein einfaches Spritzteil. Aufgrund der mechanisch einfachen Ausbildung der Dosiereinrichtung arbeitet diese auch über lange Zeiträume hinweg störungsfrei.

Mit der Weiterbildung der Erfindung gemäß Anspruch 2 wird erreicht, daß das Zuführen von Desinfektionsmittel von der Vorratsflasche zur Meßbohrung einfach durch Schwerkraft erfolgt.

Dabei wird gemäß Anspruch 3 die Abgabe von Desinfektionsmittel von der Vorratsflasche automatisch beendet, wenn die Meßbohrung vollgelaufen ist.

Die Weiterbildung der Erfindung gemäß Anspruch 4 ist im Hinblick auf eine besonders einfache Bedienung der Dosiereinrichtung von Vorteil, da das Ingangsetzen der Dosiereinrichtung und der das Desinfektionsmittel zum Versprühen fördernden Pumpe durch einen einzigen Betätigungsvorgang erfolgt.

Die Weiterbildung der Erfindung gemäß Anspruch 5 hat den Vorteil, daß das Gerät keinen elektrischen Anschluß benötigt. Druckluft, wie sie gemäß Anspruch 5 zum Absaugen des Desinfektionsmittels aus der Dosiereinrichtung verwendet wird, steht in zahnärztlichen Praxen und Labors sowieso zur Verfügung. Die im Anspruch 5 angegebene Ausbildung der Desinfektionsmittelpumpe ist auch im Hinblick auf eine möglichst feine Zerstäubung des Desinfektionsmittels von Vorteil, damit auch im Hinblick auf effektive Ausnutzung des Desinfektionsmittels und geringen Desinfektionsmittelverbrauch.

Die Weiterbildung der Erfindung gemäß Anspruch 6 bringt einen sehr kompakten Aufbau des Sprühkopfes und eine besonders effektive Zerstäubung des Desinfektionsmittels nahe beim Anwendungsort.

Bei einem Gerät gemäß Anspruch 7 ist auf einfache Weise die Forderung nach einer Freifallstrecke für das zum Abspülen des Desinfektionsmittels verwendeten Wassers realisiert.

Bei einem Gerät gemäß Anspruch 8 fließt bei verstopftem Abfluß des Behälters das überlaufende Wasser zunächst in das die Vorratsflasche aufnehmende Behälterabteil. Das Austreten von Flüssigkeit oder das Ansteigen des Flüssigkeitspegels in diesem Abteil warnt den Benutzer vor weiterer Überschwemmung. Diese Warnung kann auch automatisch erfolgen, indem man in dem die Vorratsflasche aufnehmenden Behälterabteil einen Schwimmerschalter anbringt, der auf eine optische und/oder akustische Alarmeinrichtung arbeitet.

Die Weiterbildung der Erfindung gemäß Anspruch 9 ist im Hinblick auf besonders einfache Steuerung der verschiedenen Arbeitsvorgänge innerhalb eines Desinfektionszyklus durch eine Hand des Benutzers von Vorteil. Die andere Hand bleibt dann frei, um das zu desinfizierende Objekt vor dem Sprühkopf zu bewegen.

Bei einem Gerät gemäß Anspruch 10 kann man die Steuerhebel auch gut und sicher mit einem feuchten Gummihandschuh bewegen.

Die Weiterbildung der Erfindung gemäß Anspruch 11 ist im Hinblick auf eine kostengünstige Herstellung der Ventilbank unter Einbeziehung der Gehäuseabdeckung des Behälters von Vorteil.

Bei einem Gerät gemäß Anspruch 12 erfolgt das Desinfizieren, das Abspülen des Desinfektionsmittels mit Wasser und das Wegblasen des Wassers vom desinfizierten Objekt unter geometrisch vergleichbaren Bedingungen. Das zu desinfizierende Objekt kann somit für alle drei Arbeitsgänge in gleicher Weise unter dem Sprühkopf bewegt werden, so daß keine intensive Sichtkontrolle und keine unterschiedlichen Bewegungsabläufe notwendig sind.

Die Weiterbildung der Erfindung gemäß Anspruch 13 ist im Hinblick auf die Anordnung der verschiedenen Spühdüsen auf kleinem Raum von Vorteil.

Bei einem Gerät gemäß Anspruch 14 hat man eine besonders effektive Zerstäubung des Sprühwassers.

Bei einem Gerät gemäß Anspruch 15 kann man die Düsen des Sprühkopfes sehr einfach reinigen und den Sprühkopf nach der Reinigung auch wieder einfach zusammensetzen.

Mit der Weiterbildung der Erfindung gemäß Anspruch 16 wird erreicht, daß die Düsenbohrungen der Sprühdüsen von der Beschickungsseite her frei zugänglich sind, was im Hinblick auf ein gründliches und einfaches Reinigen vorteilhaft ist.

Bei einem Gerät gemäß Anspruch 17 lassen sich die verschiedenen fluidisch aktiven Teile des Sprühkopfes auf besonders einfache Weise derart miteinander verbinden, daß der Auseinanderbau und der Zusammenbau ohne Werkzeug möglich ist, trotzdem ein sicherer Zusammenhalt der Sprühkopfteile gewährleistet ist.

Nachstehend wird die Erfindung anhand von Ausführungsbeispielen unter Bezugnahme auf die Zeichnung näher erläutert. In dieser zeigen:
- Figur 1:: eine perspektivische Explosionsdarstellung der wichtigsten Gehäuseteile und Funktionsteile eines Gerätes zur Desinfektion dentaler Objekte;

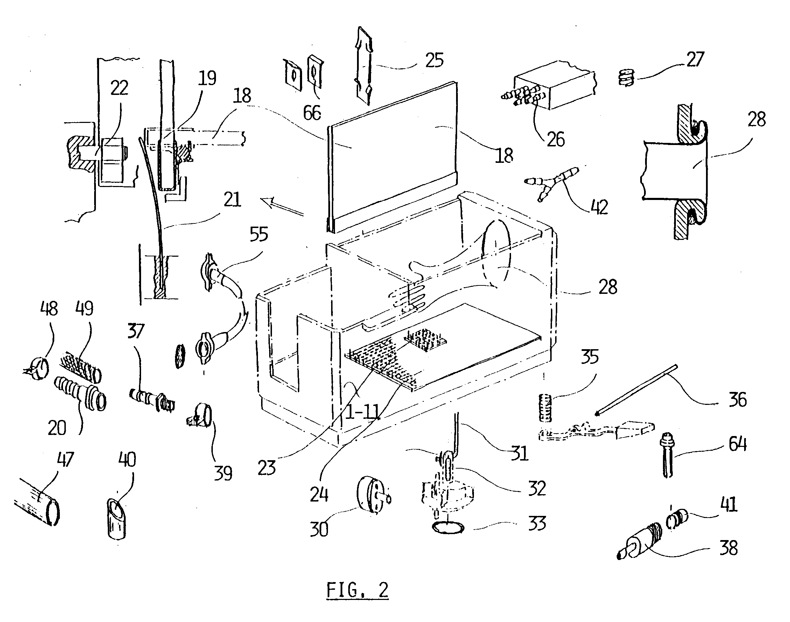

- Figur 2:: eine zweite perspektivische Explosionsdarstellung, in welcher weitere Bauelemente des Desinfektionsgerätes wiedergegeben sind, wobei das Gehäuse nur gestrichelt eingetragen ist (die Figuren 1 und 2 sind an sich zusammen zu sehen, wurden nur der besseren Darstellbarkeit halber getrennt);
- Figur 3:: eine Aufsicht auf das Gehäuse des Desinfektionsgerätes nach den Figuren 1 und 2;

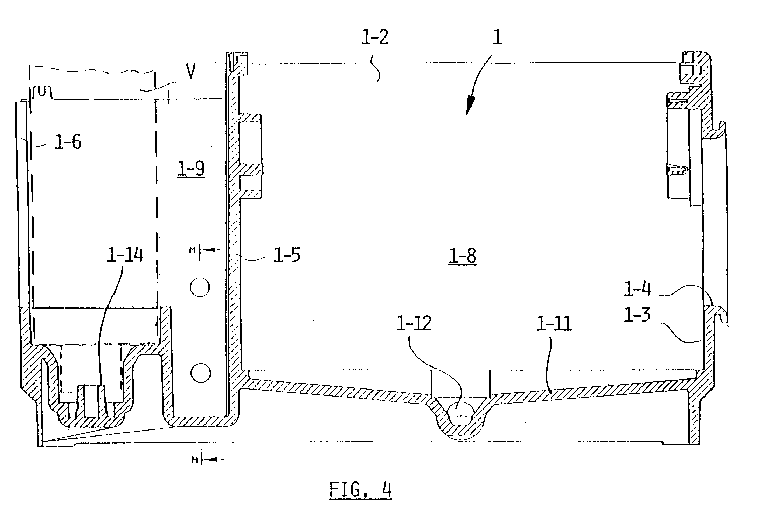

- Figur 4:: einen Längsschnitt durch das in Figur 3 gezeigte Gehäuse längs der abgewinkelten Schnittlinie A-A von Figur 3;

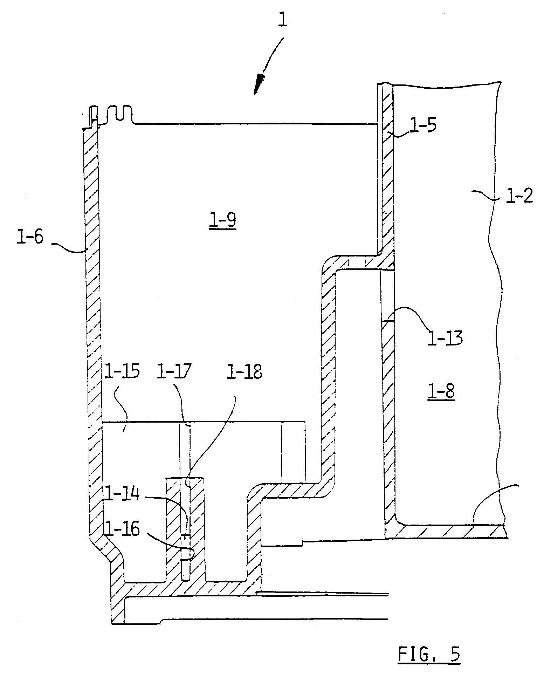

- Figur 5:: einen Schnitt durch das Gehäuse von Figur 3 längs der dortigen abgewinkelten Schnittlinie D-D;

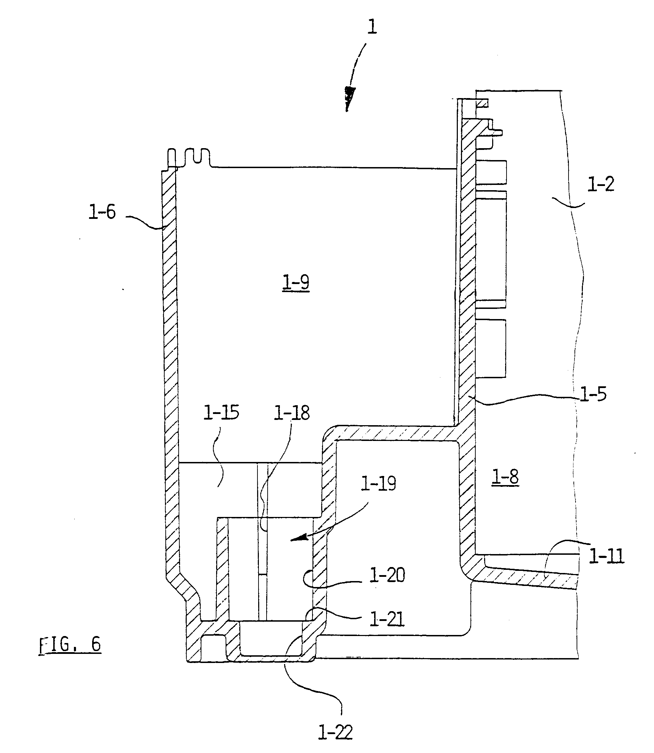

- Figur 6:: einen Schnitt durch das Gehäuse von Figur 3 längs der dortigen Schnittlinie E-E;

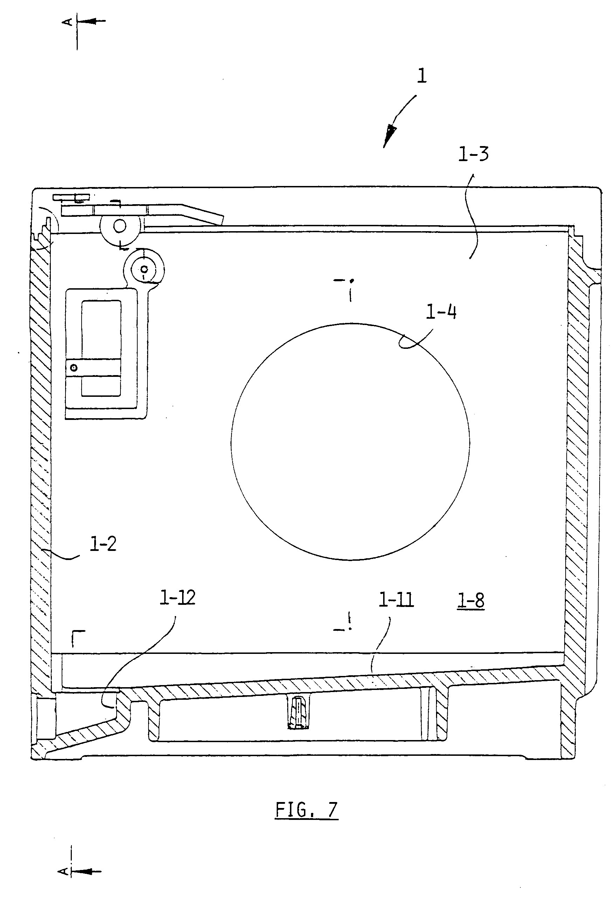

- Figur 7:: einen transversalen Schnitt durch die Mitte des Gehäuses von Figur 3;
- Figur 8:: die Hauptansichten einer Dosierglocke des Desinfektionsgerätes;
- Figur 9:: eine Aufsicht auf die Unterseite eines Sprühkopfunterteiles;
- Figur 10:: eine Aufsicht auf die in Figur 9 links gelegene Stirnseite des Sprühkopfunterteiles;
- Figur 11:: einen Schnitt durch das Sprühkopfunterteil längs der Schnittlinie A-A von Figur 10;
- Figur 12:: einen Schnitt durch das Sprühkopfunterteil längs der Schnittlinie B-B von Figur 10;
- Figur 13:: einen Schnitt durch das Sprühkopfunterteil längs der Schnittlinie C-C von Figur 10;
- Figur 14:: einen Schnitt durch das Sprühkopfunterteil längs der Schnittlinie D-D von Figur 9;
- Figur 15:: einen Schnitt durch das Sprühkopfunterteil längs der Schnittlinie E-E von Figur 10;
- Figur 16:: eine Aufsicht auf die eine Hälfte des Sprühkopfunterteiles;
- Figur 17:: eine Aufsicht auf die Unterseite eines Sprühkopfoberteiles;
- Figur 18:: eine seitliche Ansicht des Sprühkopfoberteiles;

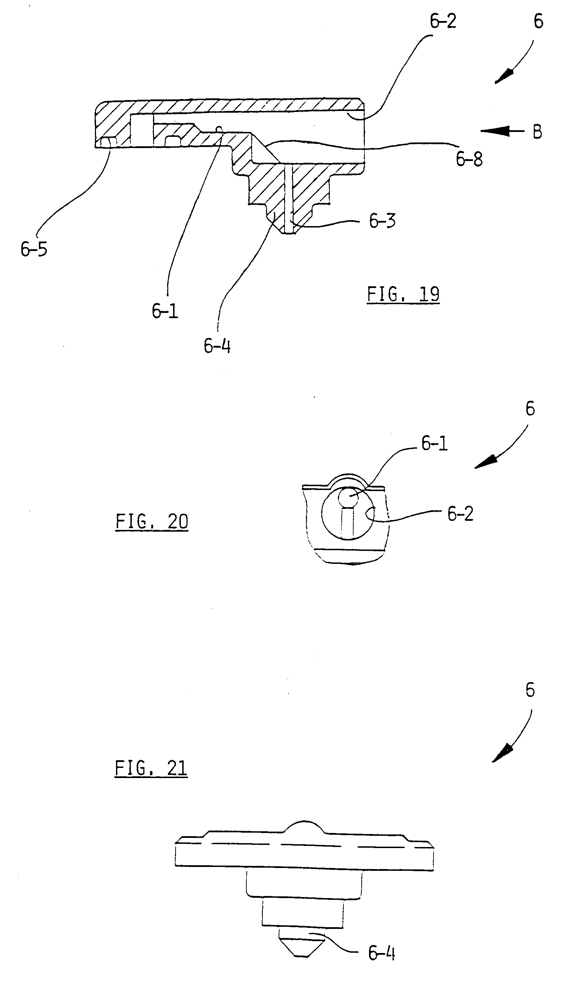

- Figur 19:: einen axialen Schnitt durch das Sprühkopfoberteil längs der Schnittlinie A-A von Figur 17;
- Figur 20:: eine Teilaufsicht auf das in Figur 19 rechts gelegene Ende des Sprühkopfoberteiles längs des dortigen Pfeiles B;
- Figur 21:: eine Aufsicht auf die in Figur 18 links gelegene Stirnfläche des Sprühkopfoberteiles;
- Figur 22:: eine Aufsicht auf die Oberseite einer Klemmkappe, welche Sprühkopfunterteil und Sprühkopfoberteil lösbar zusammenhält;
- Figur 23:: einen axialen Schnitt durch die in Figur 22 gezeigte Klemmkappe;
- Figur 24:: eine Aufsicht auf die in den Figuren 22 und 23 links gelegene Stirnfläche der Klemmkappe;
- Figur 25:: einen horizontalen Schnitt durch die Klemmkappe längs der Schnittlinie A-A von Figur 24;
- Figur 26:: eine Aufsicht auf die Unterseite eines Deckelteiles des Desinfektionsgerätes;

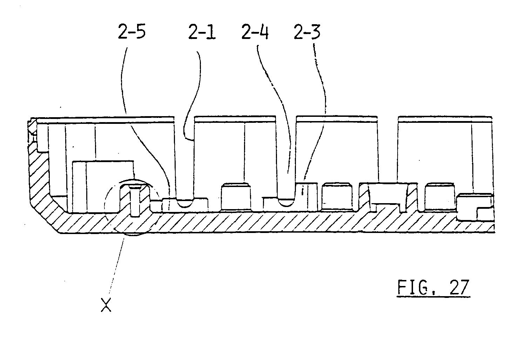

- Figur 27:: einen Schnitt durch das Deckelteil von Figur 26 längs der dortigen Schnittlinie A-A;
- Figur 28:: eine Aufsicht auf die Oberseite einer Lagerplatte, die zum schwenkbaren Lagern von Quetschventil-Steuerhebeln dient und zusammen mit der Unterseite des Deckelteiles nach den Figuren 26 und 27 Schlauchführungen vorgibt;
- Figur 29:: eine Stirnansicht der Lagerplatte nach Figur 28;
- Figur 30:: eine Aufsicht auf die Unterseite der Lagerplatte von Figur 28;
- Figur 31:: einen Schnitt durch die Lagerplatte längs der Schnittlinie A-A von Figur 30; und
- Figur 32:: eine schematische Darstellung eines Schrauben-Zerstäubereinsatzes des Sprühkopfes.

Das in der Zeichnung wiedergegebene Desinfektionsgerät für dentale Objekte, insbesondere Abdrücke, wie sie zur Anfertigung von Zahnersatz und Prothesen benötigt werden, hat ein insgesamt mit 1 bezeichnetes Gehäuse. Das Gehäuse ist ein geschäumtes Kunststoffteil und hat eine Vorderwand 1-1, eine Rückwand 1-2, eine rechte Seitenwand 1-3 mit einer Öffnung 1-4, eine Zwischenwand 1-5 sowie eine linke Seitenwand 1-6.

Auf diese Weise ist das Innere des Gehäuses 1 unterteilt in eine Behandlungskammer 1-8 sowie ein Technik-Abteil 1-9.

In die linke Seitenwand 1-6 ist ein Einsatz 63 eingehängt, der einen Aufnahmeraum 63-1 für eine Vorratsflasche V für Desinfektionsmittel vorgibt.

Im Bereich des Technik-Abteiles 1-9 ist an die Vorderwand 1-1 eine Gegengreifplatte 1-10 angeformt, die als Abstützung der Finger des Benutzers beim Nachuntendrücken von zu Quetschventilen gehörenden Steuerhebeln dienen, wie später noch genauer beschrieben wird.

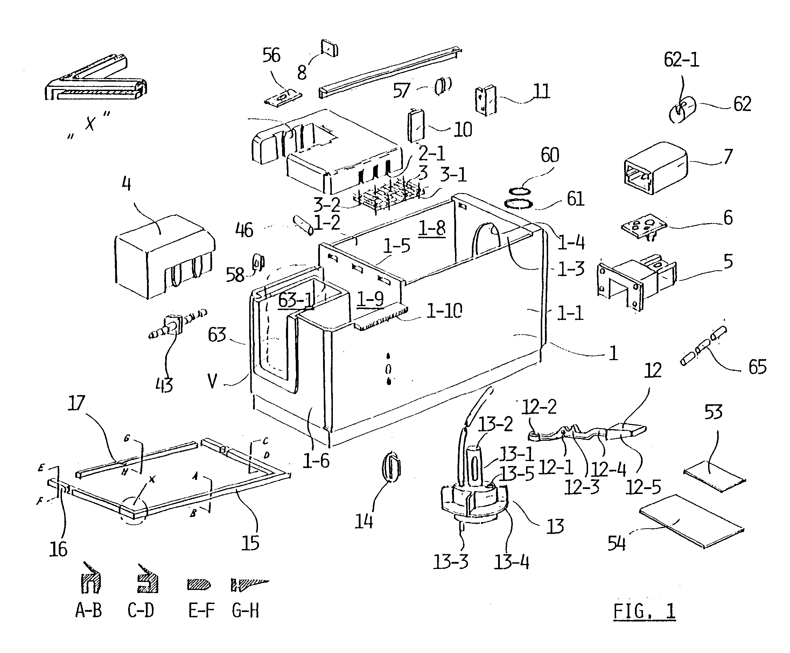

Das Technik-Abteil 1-9 ist durch einen Gehäusedeckel 2 verschlossen. Dieser ist auf seiner Unterseite mit später noch genauer zu beschreibenden Positioniernuten für zu steuernde Druckmittelschläuche versehen, die zusammen mit fluchtenden halbkreisförmigen Nuten 3-1, die in der Oberseite einer Lagerplatte 3 vorgesehen sind, im wesentliche zylindrische Positionierräume für Ventilschläuche 46 vorgeben. Die Lagerplatte 3 wird auf die Unterseite des Gehäusedeckels 2 aufgeschraubt und trägt auf ihrer Unterseite später noch genauer zu beschreibende Lageraugen für Quetschventil-Steuerhebel 12. Letztere haben ein Lagerauge 12-1, mit welchem sie auf einer Achse 36 schwenkbar gelagert sind, die in den Lageraugen auf der Unterseite der Lagerplatte 3 festgelegt ist. Am Figur 1 links gelegenen Ende tragen die Steuerhebel einen Federsitz 12-2, an welchem jeweils eine Schraubendruckfeder 35 angreift. Letztere ist über eine Einstellschraube 64 am Gehäusedeckel 2 abgestützt. Die Steuerhebel 12 haben ferner jeweils einen Quetschabschnitt 12-3, der durch eine zugeordnete Öffnung 3-2 der Lagerplatte 3 zum zu steuernden Schlauch bewegt werden kann und an diesem über ein den Veschleiß des Ventilschlauches herabsetzendes in der Öffnung 3-2 geführtes Rollstück 65 angreift. Ein Betätigungsabschnitt 12-4 der Steuerhebel 12 ist verhältnismäßig lang und erstreckt sich durch einen zugeordneten Schlitz 2-1 des Gehäusedeckels. Am in Figur 1 rechts gelegenen Ende trägt der Steuerhebel 12 einen Tastenkörper 12-5, so daß man beim zusammengebauten Gerät vor dem Gehäusedeckel 2 drei nebeneinander liegende Tasten hat, die zur Steuerung der Desinfektionsmittelzerstäubung, des Zersprühens von Wasser und des Abblasens des Objektes mit Luft dienen.

Der Gehäusedeckel 2 hat eine Ausnehmung 2-2, durch welche das innere des Aufnahmeraumes 63-1 zugänglich ist. Die Ausnehmung 2-2 ist durch einen Deckel 4 verschließbar, der formschlüssig in den Gehäusedeckel 2 einführbar ist und in der Schließstellung durch einen Rastknopf 8 verriegelbar ist.

Der obere Rand der die Behandlungskammer 1-8 umgebenden Wände ist mit Dichtungsprofilen 15, 16, 17 versehen, welche die aus Figur 1 ersichtlichen unterschiedlichen Querschnitte haben. Die Dichtungsprofile 15 bis 17 arbeiten mit der Unterseite einer Abdeckplatte 18 zusammen, deren hinteres Ende in einer Schiene 9 gehalten ist. Die Schiene 9 ist ihrerseits durch Bolzenscharniere 22 an der Seitenwand 1-3 und der Zwischenwand 1-5 gelagert, wobei mit der Rückseite der Schiene 9 eine Blattfeder 21 zusammenarbeitet, um die Abdeckplatte 18 in der Schließstellung und der Offenstellung zu verrasten.

Zum Zerstäuben von Desinfektionsmittel, zum Versprühen von Wasser und zum Abgeben von Blasluft in der Behandlungskammer 1-8 ist ein Sprühkopf vorgesehen, der ein Sprühkopfunterteil 5, ein Sprühkopfoberteil 6 sowie eine diese beiden Teile lösbar verklammernde Klemmkappe 7 aufweist.

Durch denjenigen der Steuerhebel 12, welcher das Zerstäuben von Desinfektionsmittel steuert, wird über ein Gestänge 31 eine Dosierglocke 13 bewegt. Das untere Ende des Gestänges 31 ist abgewinkelt, wie bei 31-1 gezeigt. Der abgewinkelte Endabschnitt 31-1 des Gestänges 31 erstreckt sich durch einen Längsschlitz 13-1 in einem oberen stabförmigen Betätigungsabschnitt 13-2 der Dosierglocke 13. Er arbeitet hier gegen eine Schraubendruckfeder 32. Auf diese Weise erhält man eine Totgangverbindung zwischen dem Gestänge 31 und der Dosierglocke 13. Die Sicherung des des Gestänges 31 am Betätigungsabschnitt 13-2 besorgt ein Sprengring 34.

Die Dosierglocke 13, die später noch genauer beschrieben wird, hat einen nach unten überstehenden Saugstutzen 13-3, auf welchen ein Schlauch 44 aufgesetzt ist, über den das Desinfektionsmittel dem Sprühkopf zugeführt wird. Auf die Unterseite der Dosierglocke 13 ist ein O-Ring 33 aufgesetzt.

In das Technik-Abteil 1-9 ist bei der Vorderwand 1-1 eine Kurzzeituhr 30 eingebaut, welche von der Gerätevorderseite her durch einen Drehknopf 14 auf die gewünschte Desinfektionszeit eingestellt werden kann.

Über einem im Boden des Gehäuses 1 vorgesehenen Abfluß ist ein Sieb 23 angeordnet. Über diesem Sieb und über der Bodenwand des Gehäuses 1 liegt eine gelochte Bodenplatte 24, auf der desinfizierte Objekte nach dem Abbrausen und Abblasen zum weiteren Abtropfen abgestellt werden können.

In die Öffnung 1-4 in der rechten Seitenwand 1-3 des Gehäuses 1 ist ein aus Gummi hergestellter Handschuh 28 eingeknöpft und durch einen O-Ring 29 gesichert.

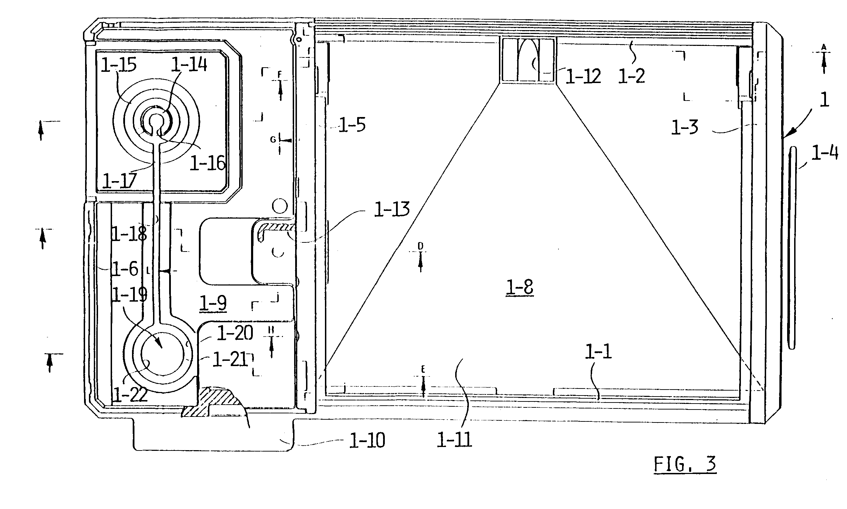

Wie aus Figur 3 ersichtlich, hat die Bodenwand 1-11 des Gehäuses 1 eine Ablauffläche, die zu einer Ablauföffnung 1-12 hinführt. In der Zwischenwand 1-5 ist ein Überlauf 1-13 vorgesehen, der in das Technik-Abteil 1-9 führt.

Die Bodenwand des Technik-Abteiles trägt im Inneren des Aufnahmeraumes 63-1 einen Dorn 1-14, der zum Durchstoßen einer Versiegelungsfolie der Vorratsflasche V dient. Der Dorn 1-14 ist von einer Ringwand 1-15 umgeben und begrenzt so einen Ringraum, in welchem der Hals der Vorratsflasche V Aufnahme findet.

Der Dorn 1-14 und die Ringwand 1-15 sind geschlitzt, wie bei 1-16 bzw. 1-17 gezeigt. In die Bodenwand ist eine transversale Rinne 1-18 eingearbeitet, welche in eine Meßbohrung 1-19 führt.

Die Meßbohrung 1-19 hat einen oberen, mit der Rinne 1-18 in Verbindung stehenden Bohrungsabschnitt 1-20 sowie einen hiervon durch eine Schulter 1-21 getrennten unteren Bohrungsabschnitt 1-22.

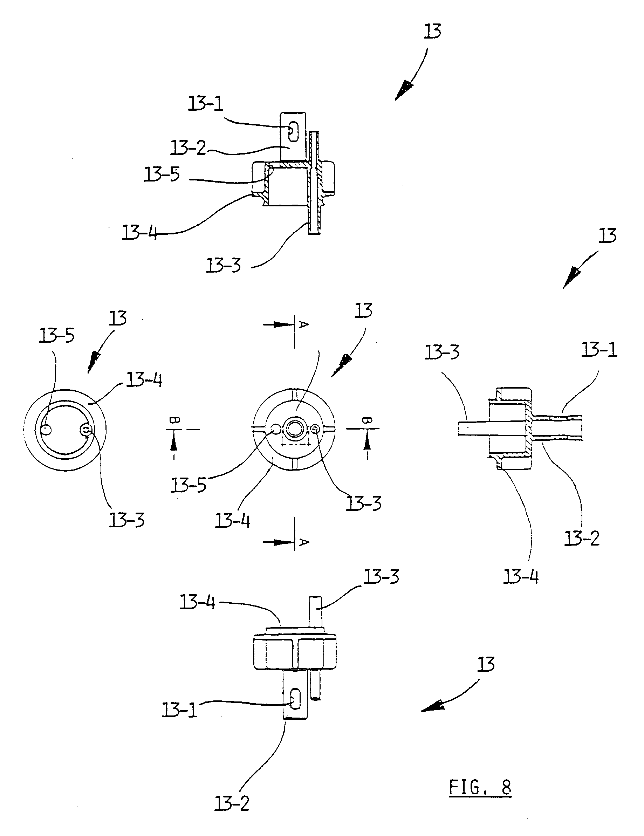

Wie aus Figur 8 ersichtlich, hat die Dosierglocke 13 einen Flanschabschnitt 13-4, der in den oberen Bohrungsabschnitt 1-20 der Meßbohrung 1-19 hineinbewegbar ist. An der Unterseite des Flansches 13-4 liegt der O-Ring 33 an. Wird die Dosierglocke 13 durch Betätigen des Desinfektionsmittel-Steuerhebels über das Gestänge 31 nach unten bewegt, so kommt der O-Ring 33 in Anlage an die Schulter 1-21 und der untere Bohrungsabschnitt 1-22 wird dicht verschlossen. Damit kann in diesen Bohrungsabschnitt kein Desinfektionsmittel mehr über die Rinne 1-18 nachfließen. Das dann in der Meßbohrung eingeschlossene Desinfektionsmittelvolumen kann über den Saugstutzen 13-3 vom Sprühkopf abgesaugt werden, wobei Luft über eine Entlüftunsöffnung 13-5 ins Glockeninnere strömt.

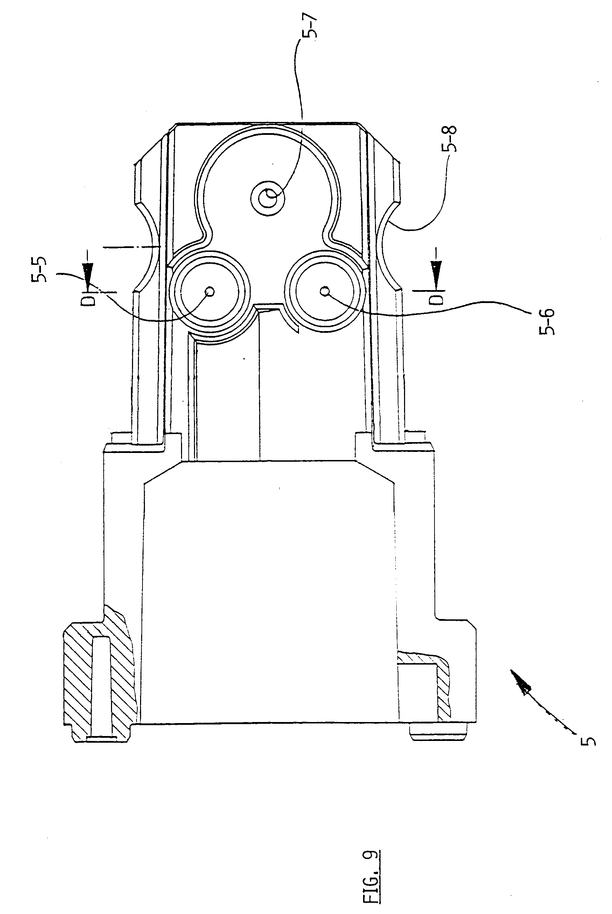

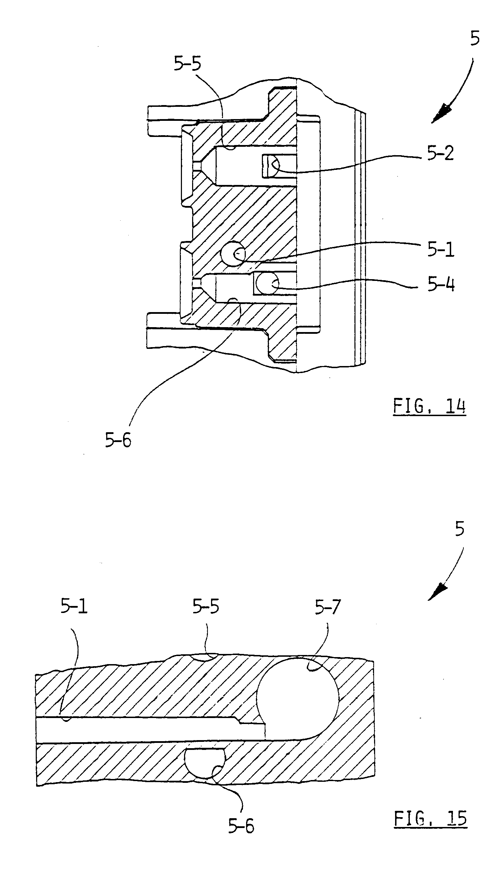

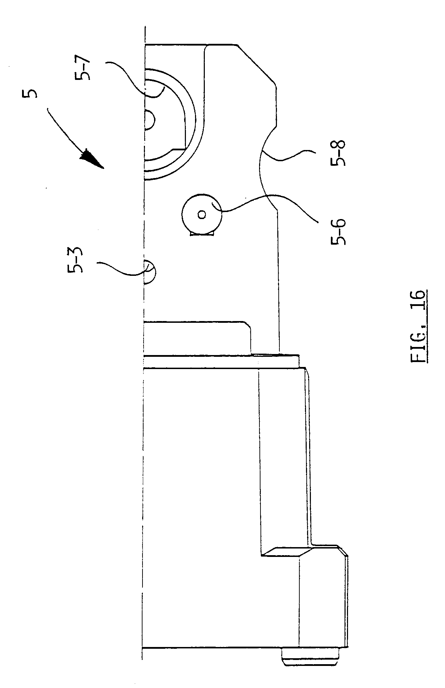

Nunmehr werden unter Bezugnahme auf die Figuren 9 bis 16 Einzelheiten des Sprühkopfunterteiles beschrieben.

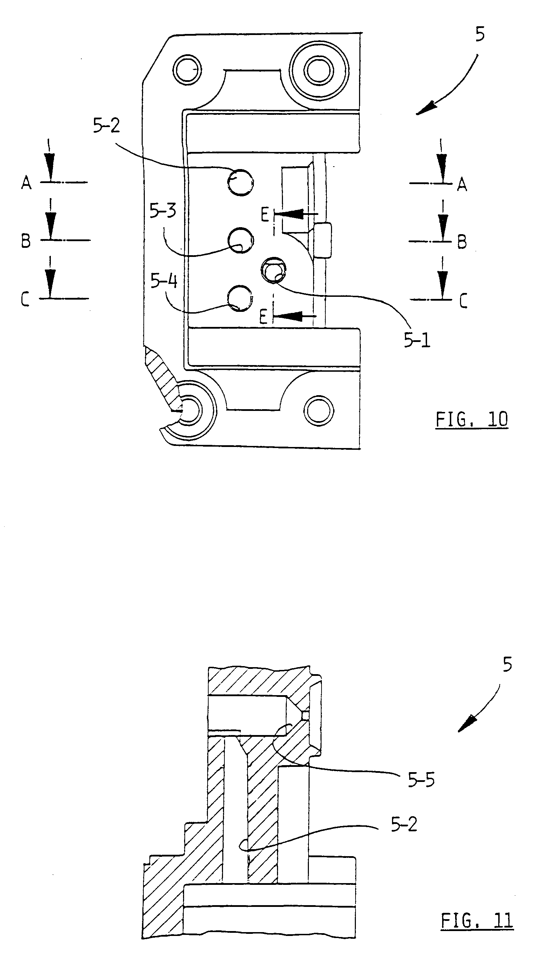

Wie aus Figur 10 ersichtlich, hat das an der Zwischenwand 1-5 oberhalb des Überlaufes 1-13 befestigte Sprühkopfunterteil 5 vier in seine in Figur 10 obenliegende, zur Zwischenwand 1-5 ausmündende Kanäle, nämlich einen Desinfektionsmittelkanal 5-1, einen Wasserkanal 5-2, einen Zerstäubungsluftkanal 5-3 und einen Blasluftkanal 5-4.

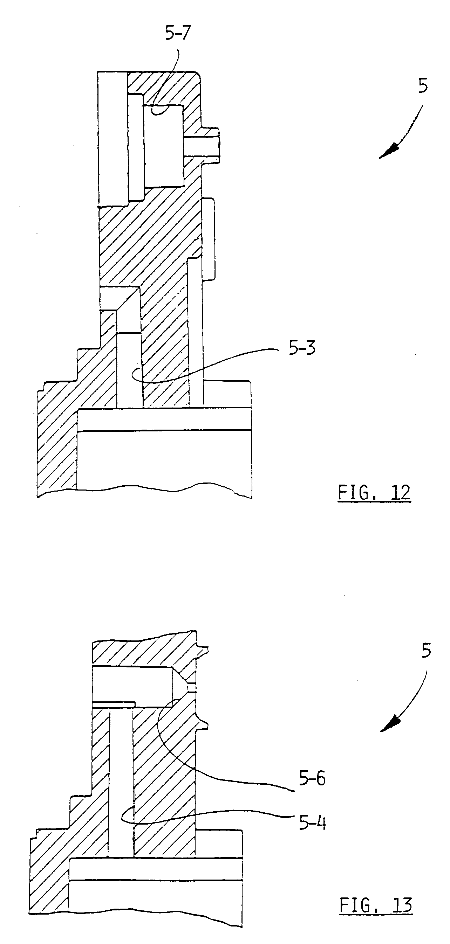

Wie aus den Figuren 11 und 13 ersichtlich, münden der Wasserkanal 5-2 und der Blasluftkanal 5-4 direkt in Düsenbohrungen 5-5 und 5-6, die zu Reinigungszwecken zur Oberseite des Sprühkopfunterteiles hin offen sind, bei betriebsbereitem Sprühkopf jedoch durch das Sprühkopfoberteil 6 verschlossen werden.

In die Wasser-Düsenbohrung 5-5 ist bei zusammengebautem Sprühkopf der Schrauben-Zerstäuber 27 eingesetzt. Man erhält so insgesamt einen weichen und weiten Wasservorhang.

Der Zerstäubungsluftkanal 5-3 ist abgewinkelt und mündet in die Oberseite des Sprühkopfunterteiles 5 aus. Von dort tritt die Zerstäubungsluft in einen Luftkanal 6-1 des Sprühkopfoberteils ein, der zu einer Sackbohrung 6-2 führt. In die Sackbohrung 6-2 ist bei betriebsbereitem Sprühkopf ein in Figur 1 gezeigter Stopfen 62 eingesetzt, der mit einer schrägen Nut 62-1 versehen ist, die zusammen mit einer schrägen Rippe 6-8 am Boden der Sackbohrung 6-2 einen Kanal begrenzt. Das freie Ende der Sackbohrung 5-2 ist durch den Stopfen 62 verschlossen.

In die Sackbohrung 6-2 mündet in radialer Richtung ein Düsenkanal 6-3, der in einem kegelstumpfförmigen Düsenkopf 6-4 ausläuft.

Der Düsenkopf 6-4 ist seinerseits unter radialem Spiel in eine Injektorkammer 5-7 des Sprühkopfunterteiles 5 einfahrbar, so daß man zwischen dem Düsenkopf 6-4 und der Wand der Injektorkammer 5-7 einen Ringraum erhält. In diesen Ringraum mündet in tangentialer Richtung der Desinfektionsmittelkanal 5-1.

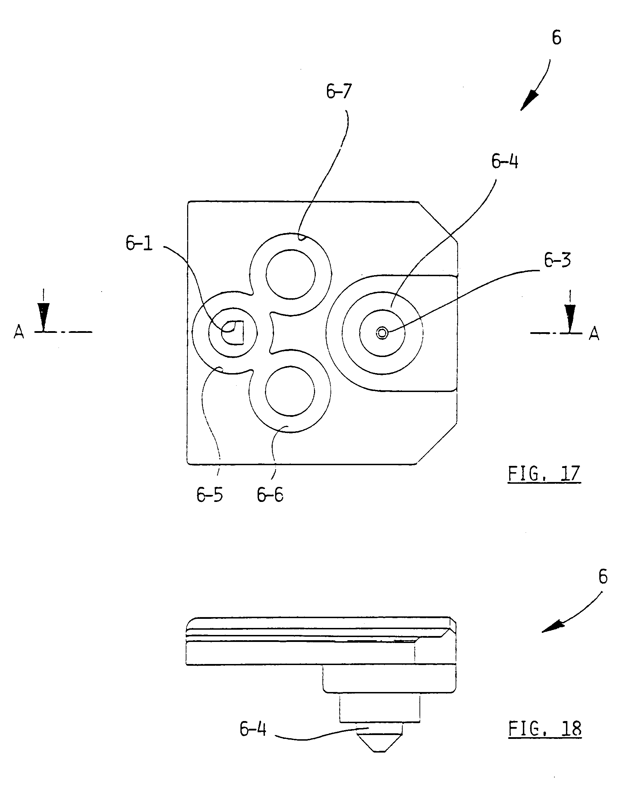

Wie aus Figur 17 ersichtlich, sind in die Unterseite des Sprühkopfoberteiles Ringnuten 6-5, 6-6, 6-7 eingestochen, in welche O-Ringe eingelegt werden (nicht gezeigt). Hierdurch wird eine dichte Verbindung zwischen dem Blasluftkanal 5-4 und dem Luftkanal 6-1 hergestellt und werden die Enden der Düsenbohrungen 5-5 und 5-6 abgedichtet.

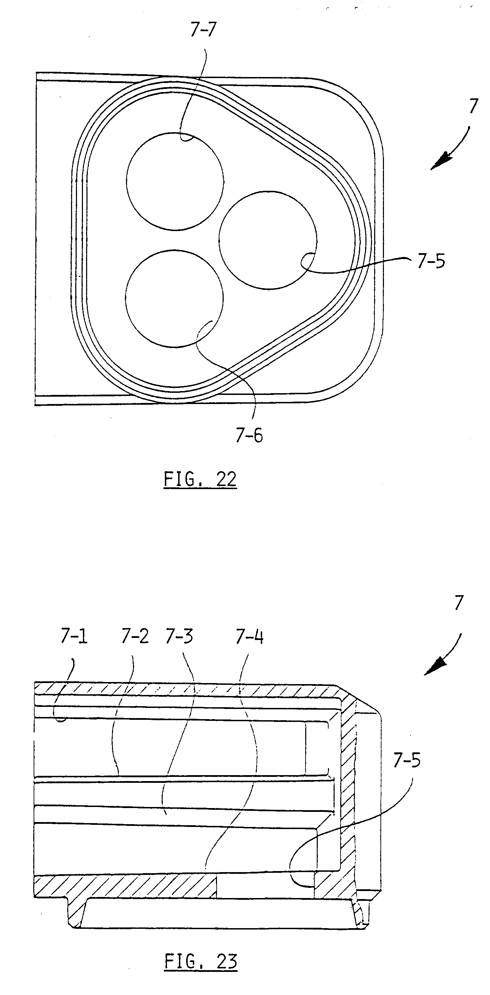

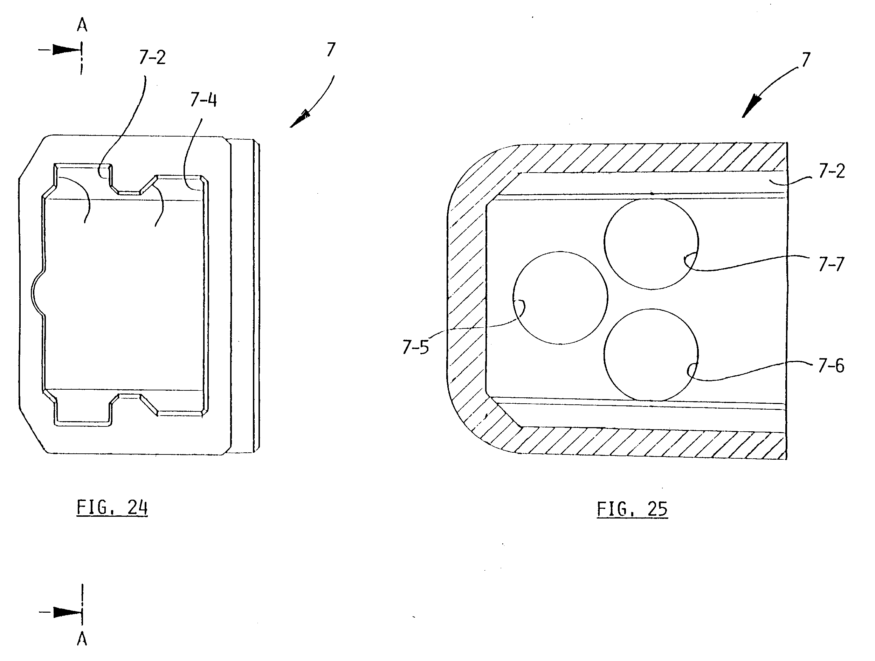

Das Sprühkopfoberteil 6 wird zum Zusammenbau des Sprühkopfes von oben auf das Sprühkopfunterteil 5 aufgesetzt, derart, daß der Düsenkopf 6-4 in die Injektorkammer 5-7 zu liegen kommt, wie oben beschrieben. In dieser Stellung werden Sprühkopfunterteil und Sprühkopfoberteil durch die Klemmkappe 7 fixiert, von welcher Einzelheiten in den Figuren 22 bis 25 gezeigt sind.

Die Klemmkappe 7 hat Führungsflächen 7-1, 7-2, 7-3 und 7-4, die mit komplementären Führungsflächen auf dem Sprühkopfunterteil 5 und dem Sprühkopfoberteil 6 zusammenarbeiten. Wie aus Figur 23 ersichtlich, sind die Führungsflächen 7-1 bis 7-4 leicht keilförmig angestellt, um eine Selbsthemmung zu erzielen. In ihrer Unterseite ist die Klemmkappe mit drei Öffnungen 7-5, 7-6 und 7-7 versehen, deren Achsen mit den Achsen der Düsenbohrungen 5-5 und 5-6 bzw. des Düsenkopfes 6-4 fluchten. Die Öffnungen 7-5, 7-6 und 7-7 lassen das versprühte Desinfektionsmittel, das zersprühte Wasser und die Blasluft unbehindert durchtreten.

Um das Sprühkopfoberteil 6 leichter vom Sprühkopfunterteil 5 abnehmen zu können, haben die Seitenflächen des Sprühkopfunterteiles kreisförmige Ausnehmungen 5-8.

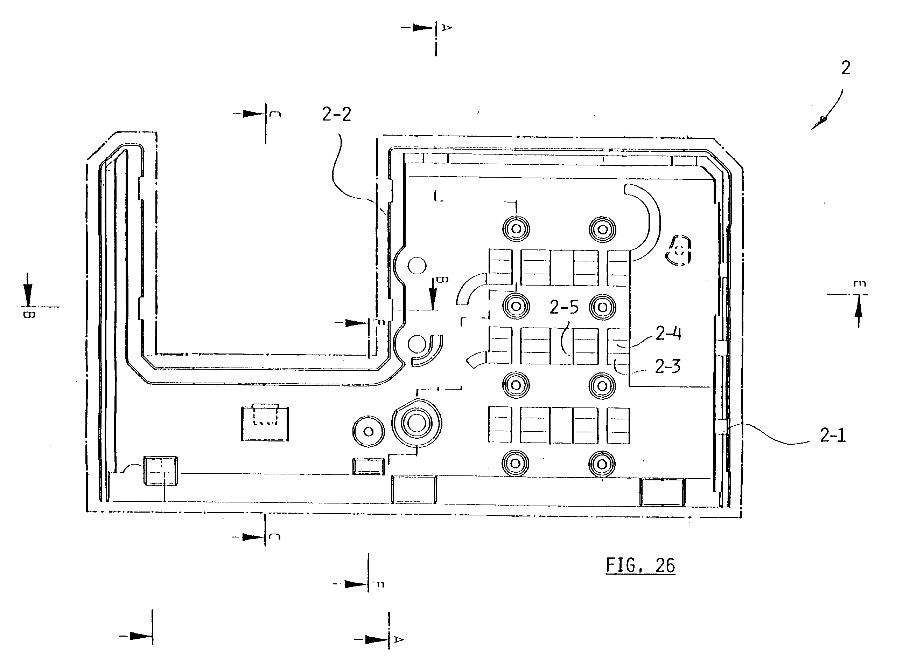

Wie aus Figur 26 ersichtlich, sind an die Unterseite des Gehäusedeckels 2 drei Positionierrippen 2-3 angeformt, die jeweils eine halbkreisförmige Positioniernut 2-4 vorgeben, welche zusammen mit einer fluchtenden Nut 3-1 der Lagerplatte 3 zur seitlichen Fixierung eines Schlauches 46 dienen, über den Zerstäubungsluft, Blasluft oder Wasser dem Sprühkopf zugeführt wird.

Die Positionierrippen 2-3 haben eine mittige Rippenausnehmung 2-5, in welche der Quetschabschnitt 12-3 eines zugeordneten Steuerhebels 12 einfahren und auf das dort darunterliegende Rollstück 65 drücken kann, wobei der zugehörige Schlauch 46 zusammengequetscht wird.

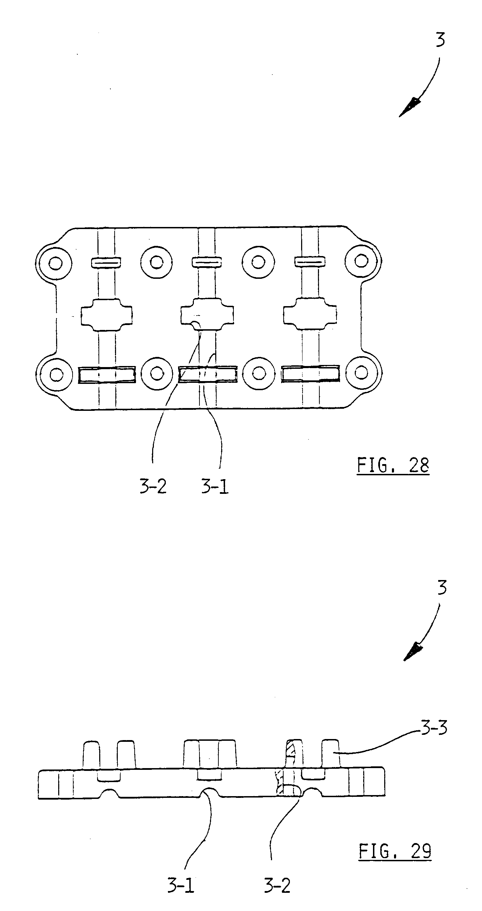

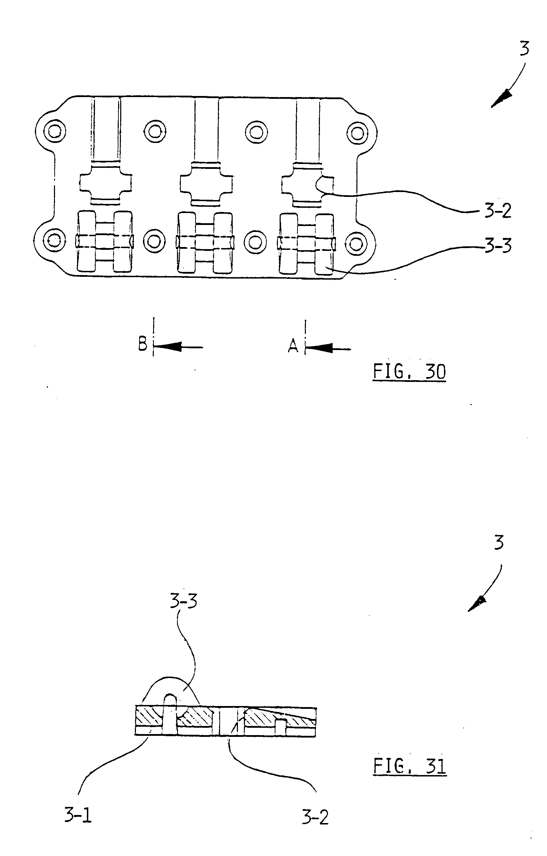

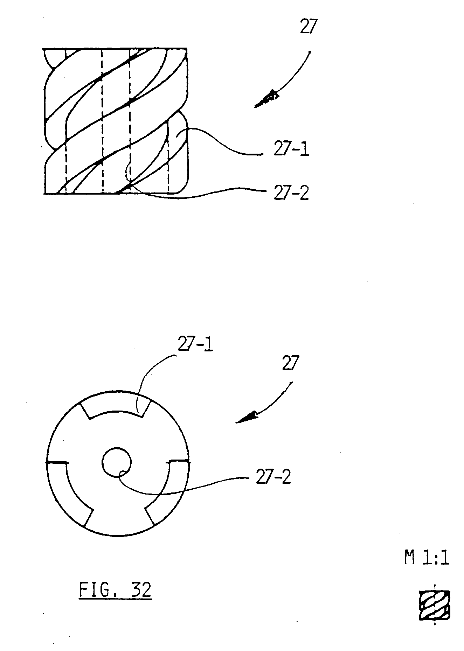

Wie aus den Figuren 28 bis 31 ersichtlich, hat die Lagerplatte 3 neben den schon erwähnten Nuten 3-1 und Öffnungen 3-2 auf ihrer Unterseite Lageraugen 3-3, in welchen die Achse 36 Aufnahme findet. Die Steuerhebel 12 sind durch die Schraubendruckfedern 35 entgegen dem Uhrzeigersinne vorgespannt, so daß ihr Quetschabschnitt 12-3 in die zugeordnete Öffnung 3-2 der Lagerplatte 3 und über das Rollteil 65 auf den zugehörigen Schlauch 46 drückt. Dieser ist somit normalerweise geschlossen und läßt keine Zerstäubungsluft, keine Blasluft und kein Wasser hindurch. Die hinter den Quetschstellen liegenden Enden der drei gesteuerten Schläuche sind mit dem Einlaß des Zerstäubungsluftkanales 5-3, des Wasserkanales 5-2 und des Blasluftkanales 5-4 des Sprühkopfunterteiles verbunden. Durch Drücken eines der Tastkörper 12-5 kann man somit das Desinfizieren, das Abspülen und das Trockenblasen eines zu desinfizierenden Gegenstandes einleiten, wobei das Freigeben des Schlauches 46 für die Zerstäubungsluft zugleich über das Gestänge 31 zu einem Absenken der Dosierglocke 13 führt. Durch den vom Düsenkopf 6-4 in die Injektorkammer 5-7 abgegebenen scharfen Luftstrahl wird dann das Desinfektionsmittel über den Schlauch 44 aus dem nun dicht verschlossenen unteren Bohrungsabschnitt 1-22 der Meßbohrung 1-19 angesaugt. Die Zugabe von Desinfektionsmittel endet zwangsläufig, wenn der untere Bohrungsabschnitt 1-22 leergesaugt ist. Erst dann, wenn der Desinfektions-Steuerhebel 12 wieder freigegeben ist, strömt über die nun keine Flüssigkeit mehr enthaltende Rinne 1-15 neues Desinfektionsmittel aus der Vorratsflasche V. Dieser Desinfektionsmittelstrom endet, wenn der Flüssigkeitspegel in der Meßbohrung 5-5 und damit auch der Rinne 1-15 soweit angestiegen ist, daß über die geschlitzte Ringwand 1-15 keine Luft mehr ins Innere der Vorratsflasche V gelangen kann.

## Patentansprüche

1. Gerät zum Desinfizieren von dentalen Objekten wie Gebißabdrücken, mit einem wassererdichten Kasten (1, 18); mit einem von einer Kastenwand (1-5) getragenen Sprühkopf (5, 6), welcher eine Sprühdüse (5-7, 6-4) für Desinfektionsmittel aufweist, mit einer Vorratsflasche (V) für Desinfektionsmittel; ; mit einer mit der Vorratsflasche (V) in Verbindung stehenden Dosiereinrichtung (1-19, 13) und mit einer Pumpe (5-7, 6-4) zum Fördern von Desinfektionsmittel von der Dosiervorrichtung (1-19, 13) zur Desinfektionsmittel-Sprühdüse (5-7, 6-4); dadurch gekennzeichnet, daß die Dosiereinrichtung eine Meßbohrung (1-19) und ein mit dieser Zusammenarbeitendes Verschlußstück (13) aufweist.

2. Gerät nach Anspruch 1, dadurch gekennzeichnet, daß die Meßbohrung (1-19) über eine Rinne (1-18) mit dem offenen unteren Ende der Vorratsflasche (V) in Verbindung steht.

3. Gerät nach Anspruch 2, dadurch gekennzeichnet, daß das flaschenseitige Ende der Rinne (1-18) eine Aufweitung (1-15) aufweist, in welcher ein in den Flaschenhals einführbarer hohler Dorn (1-14) angeordnet ist.

4. Gerät nach einem der Ansprüche 1 bis 3, dadurch gekennzeichnet, daß das Verschlußstück (13) über ein Gestänge (31) durch einen Steuerhebel (12) in die Schließstellung bewegbar ist, durch welchen zugleich die Desinfektionsmittelpumpe (5-7, 6-4) inganggesetzt wird.

5. Gerät nach einem der Ansprüche 1 bis 4, dadurch gekennzeichnet, daß die Desinfektionsmittelpumpe einen druckluftbetriebenen Injektor (5-7, 6-4) aufweist.

6. Gerät nach Anspruch 5, dadurch gekennzeichnet, daß der Injektor (5-7, 6-4) Teil der Desinfektionsmittel-Sprühdüse ist.

7. Gerät nach einem der Ansprüche 1 bis 6, gekennzeichnet durch einen in einer (1-5) der Wände des Kastens (1, 18) vorgesehenen Überlauf (1-13), der unterhalb des Sprühkopfes (5, 6) liegt.

8. Gerät nach Anspruch 7, dadurch gekennzeichnet, daß die mit dem Überlauf (1-13) versehene Behälterwand eine Zwischenwand (1-5) ist, welche ein die Vorratsflasche (V) aufnehmendes Technik-Abteil (1-9) begrenzt.

9. Gerät nach einem der Ansprüche 1 bis 8, dadurch gekennzeichnet, daß ein Gehäusedeckel (2) eine Schlauchventilbank trägt, die aufweist: Positioniernuten (2-4, 3-1) zum nebeneinanderliegenden Positionieren einer Mehrzahl von Ventilschläuchen (46), Augen (3-3) zum nebeneinanderliegenden Lagern einer Mehrzahl von Steuerhebeln (12), die mit den Ventilschläuchen (46) zusammenarbeiten und einen Betätigungsabschnitt (12-4) sowie einen Quetschabschnitt (12-3) haben, welcher vorzugsweise über Druckrollen (65) auf den zugeordneten Ventilschlauch arbeitet.

10. Gerät nach Anspruch 9, dadurch gekennzeichnet, daß die Betätigungsabschnitte (12-4) der Steuerhebel Tastenkörper (12-5) tragen und unter den Tastenkörpern (12-5) eine Gegengreifleiste (1-10) vorgesehen ist.

11. Gerät nach Anspruch 9 oder 10, dadurch gekennzeichnet, daß die Positioniernuten (2-4), 3-1) auf der Unterseite des Gehäusedeckels (2) bzw. der Oberseite einer die Steuerhebel-Lageraugen (3-3) tragenden Lagerplatte (3) vorgesehen sind und daß die Lagerplatte (3) Öffnungen (3-2) aufweist, durch welche die Quetschabschnitte (12-3) der Steuerhebel (12) bzw. die Druckrollen (65) hindurchbewegbar sind.

12. Gerät nach einem der Ansprüche 1 bis 11, dadurch gekennzeichnet, daß der Sprühkopf (5, 6) neben der Desinfektionsmittel-Sprühdüse (5-7, 6-4) eine Sprühdüse für Wasser (5-5) sowie eine Blasluft-Düse (5-6) aufweist.

13. Gerät nach Anspruch 12, dadurch gekennzeichnet, daß die Achsen der drei Düsen (5-7, 6-4, 5-5, 5-6) ein gleichseitiges Dreieck vorgeben.

14. Gerät nach Anspruch 12 oder 13, dadurch gekennzeichnet, daß der Wasser-Sprühdüse (5-5) ein Schrauben-Zerstäubereinsatz (27) zugeordnet ist.

15. Gerät nach einem der Ansprüche 11 bis 14, dadurch gekennzeichnet, daß ein Düsenkopf (6-4) der Desinfektionsmittel-Sprühdüse (5-7, 6-4) in einem Sprühkopfoberteil (6) angeordnet ist, welches lösbar mit dem Sprühkopfunterteil (5) verbunden ist, wobei ein Zerstäubungsluft-Speisekanal (5-4, 6-1) durch die Trennfläche zwischen Sprühkopfoberteil (6) und Sprühkopfunterteil (5) hindurchgeführt ist.

16. Gerät nach Anspruch 15, dadurch gekennzeichnet, daß Düsenbohrungen (5-5, 5-6) für Wasser bzw. Blasluft in die Oberseite des Sprühkopfunterteiles (5) ausmünden und durch darüber liegende Abschnitte des Sprühkopfoberteiles (6) dicht verschlossen sind.

17. Gerät nach Anspruch 15 oder 16, gekennzeichnet durch eine über Sprühkopfunterteil (5) und Sprühkopfoberteil (6) aufschiebbare Klemmkappe (7) mit vorzugsweise keilförmigen Führungsflächen (7-1, 7-2, 7-3, 7-4) zum Verspannen der beiden Sprühkopfteile.

## Claims

1. Device for disinfecting dental objects, such as teeth impressions, comprising a water-tight box (1; 18), a spray head (5, 6) which is carried by one wall (1-5) of the box and has a spray nozzle (5-7, 6-4) for disinfectant; a supply bottle (V) for disinfectant; a metering device (1-19, 13) which is in communication with the supply bottle (V), and a pump (5-7, 6-4) for feeding disinfectant from the metering device (1-19, 13) to the disinfectant spray nozzle (5-7; 6-4); characterised in that the metering device has a measuring bore (1-19) and a closure piece (13) which cooperates with the latter.

2. Device according to claim 1, characterised in that the measuring bore (1-19) is in communication with the open, lower end of the supply bottle (V) by way of a channel (1-18).

3. Device according to claim 2, characterised in that the end of the channel (1-18) towards the bottle has a widened portion (1-15) in which a hollow spike (1-14) which can be introduced into the neck of the bottle is arranged.

4. Device according to any one of claims 1 to 3, characterised in that the closure piece (13) is movable via a rod linkage (31) into the closed position by a control lever (12) by which the disinfectant pump (5-7, 6-4) is set in operation at the same time.

5. Device according to any one of claims 1 to 4, characterised in that the disinfectant pump has a compressed-air-operated injector (5-7, 6-4).

6. Device according to claim 5, characterised in that the injector (5-7, 6-4) is part of the disinfectant spray nozzle.

7. Device according to any one of claims 1 to 6, characterised by an overflow (1-13) which is provided in one (1-5) of the walls of the box (1, 18) and lies beneath the spray head (5, 6).

8. Device according to claim 7, characterised in that the vessel wall provided with the overflow (1-13) is a partition wall (1-5) that delimits a technical compartment (1-9) containing the supply bottle (V).

9. Device according to any one of claims 1 to 8, characterised in that a housing lid (2) carries a tube valve bank which comprises: positioning grooves (2-4, 3-1) for positioning a plurality of valve tubes (46) side by side, eyes (3-3) for supporting a plurality of control levers (12) side by side, which control levers cooperate with the valve tubes (46) and have an operating portion (12-4) and a pinching portion (12-3) that acts on the associated valve tube preferably by way of pressure rollers (65).

10. Device according to claim 9, characterised in that the operating portions (12-4) of the control levers carry key bodies (12-5), and a counter-grip ledge (1-10) is provided under the key bodies (12-5).

11. Device according to claim 9 or 10, characterised in that the positioning grooves (2-4, 3-1) are respectively provided on the underside of the housing lid (2) and on the upper side of a bearing plate (3) carrying the control lever bearing eyes (3-3), and the bearing plate (3) has openings (3-2) through which the pinching portions (12-3) of the control levers (12) and the pressure rollers (65) can be moved.

12. Device according to any one of claims 1 to 11, characterised in that the spray head (5, 6) has, in addition to the disinfectant spray nozzle (5-7, 6-4), a spray nozzle for water (5-5) and an air-blowing nozzle (5-6).

13. Device according to claim 12, characterised in that the axes of the three nozzles (5-7, 6-4, 5-5, 5-6) form an equilateral triangle.

14. Device according to claim 12 or 13, characterised in that a helical atomizer insert (27) is associated with the water spray nozzle (5-5).

15. Device according to any one of claims 11 to 14, characterised in that a nozzle head (6-4) of the disinfectant spray nozzle (5-7, 6-4) is arranged in an upper part (6) of the spray head, which upper part is separably connected to the lower part (5) of the spray head, wherein an atomizing air feed channel (5-4, 6-1) is passed through the parting plane between the upper part (6) of the spray head and the lower part (5) of the spray head.

16. Device according to claim 15, characterised in that nozzle bores (5-5, 5-6) for water and blowing air open into the upper side of the lower part (5) of the spray head and are tightly sealed by portions of the upper part (6) of the spray head lying thereover.

17. Device according to claim 15 or 16, characterised by a clamping cap (7) which can be pushed on over the lower part (5) of the spray head and the upper part (6) of the spray head and has preferably wedge-shaped guide surfaces (7-1, 7-2, 7-3, 7-4) for bracing the two parts of the spray head together.

## Revendications

1. Appareil pour désinfecter des articles dentaires, tels que des porteempreintes pour prothèses dentaires, comprenant un boîtier (1, 18) étanche, une tête de pulvérisation (5, 6) portée par une paroi (1-5) du boîtier, qui présente une buse de pulvérisation (5-7, 6-4) pour l'agent désinfectant, comprenant également un dispositif de dosage (1-19, 13) relié à un flacon (V), et une pompe (5-7, 6-4) pour amener l'agent désinfectant du dispositif de dosage (1-19, 13) à la buse de pulvérisation (5-7, 6-4) pour l'agent désinfectant, caractérisé en ce que le dispositif de dosage présente un perçage de mesure (1-19) et un élément de fermeture (13) coopérant avec ce dernier.

2. Appareil selon la revendication 1, caractérisé en ce que le perçage de mesure (1-19) est relié par une rigole (1-18) à l'extrémité inférieure ouverte du flacon (V).

3. Appareil selon la revendication 2, caractérisé en ce que l'extrémité de la rigole (1-18) située du côté du flacon présente un élargissement (1-15) dans lequel est disposé un poinçon creux (1-14) pouvant être introduit dans le col du flacon.

4. Appareil selon l'une des revendications 1 à 3, caractérisé en ce que l'élément de fermeture (13) peut être déplacé en position fermée par le biais d'une tringle (31) à l'aide d'un levier de commande (12) qui actionne simultanément la pompe pour l'agent désinfectant (5-7, 6-4).

5. Appareil selon l'une des revendications 1 à 4, caractérisé en ce que la pompe pour l'agent désinfectant présente un injecteur (5-7, 6-1) fonctionnant à l'air comprimé.

6. Appareil selon la revendication 5, caractérisé en ce que l'injecteur (5-7, 6-4) fait partie de la buse de pulvérisation pour l'agent désinfectant.

7. Appareil selon l'une des revendications 1 à 6, caractérisé par un tropplein (1-13) prévu dans l'une (1-5) des parois du boîtier (1, 18), qui se situe sous la tête de pulvérisation (5, 6).

8. Appareil selon la revendication 7, caractérisé en ce que la paroi du boîtier munie du trop-plein (1-13) est une paroi intermédiaire (1-5) qui délimite la partie technique (1-9) renfermant le flacon (V).

9. Appareil selon l'une des revendications 1 à 8, caractérisé en ce qu'un couvercle (2) du boîtier porte un support de valves qui présente : des rainures de positionnement (2-4, 3-1) pour positionner plusieurs valves (46) côte à côte, des oeillets (3-3) pour monter plusieurs leviers de commande (12) côte à côte, lesquels coopèrent avec les valves (46) et comportent une partie d'actionnement (12-4) et une partie de compression (12-3) qui agit sur la valve qui lui est associée, de préférence par le biais de galets de compression (65).

10. Appareil selon la revendication 9, caractérisé en ce que les parties d'actionnement (12-4) des leviers de commande portent des touches (12-5), et en ce qu'une plaquette d'appui (1-10) est prévue sous les touches (12-5).

11. Appareil selon la revendication 9 ou 10, caractérisé en ce que les rainures de positionnement (2-4, 3-1) sont prévues sur le dessous du couvercle (2) du boîtier ou sur le dessus d'une plaque d'appui (3) portant les oeillets de montage (3-3) pour les leviers de commande, et en ce que la plaque d'appui (3) présente des ouvertures (3-2) à travers lesquelles les parties de compression (12-3) des leviers de commande (12) ou les galets de compression (65) peuvent être déplacé(e)s.

12. Appareil selon l'une des revendications 1 à 11, caractérisé en ce que la tête de pulvérisation (5, 6) présente, outre la buse de pulvérisation pour l'agent désinfectant (5-7, 6-4), une buse de pulvérisation pour l'eau (5-5) et une buse pour l'air soufflé (5-6).

13. Appareil selon la revendication 12, caractérisé en ce que les axes des trois buses (5-7, 6-4, 5-5, 5-6) forment un triangle équilatéral.

14. Appareil selon la revendication 12 ou 13, caractérisé en ce qu'un pulvérisateur à hélice (27) est associé à la buse de pulvérisation pour l'eau (5-5).

15. Appareil selon l'une des revendications 11 à 14, caractérisé en ce qu'une tête d'injecteur (6-4) de la buse de pulvérisation pour l'agent désinfectant (5-7, 6-4) est disposée dans une partie supérieure (6) de la tête de pulvérisation qui est reliée de manière amovible à la partie inférieure (5) de la tête de pulvérisation, un canal d'alimentation pour l'air de pulvérisation (5-4, 6-1) traversant la surface de séparation entre la partie supérieure (6) de la tête de pulvérisation et la partie inférieure (5) de la tête de pulvérisation.

16. Appareil selon la revendication 15, caractérisé en ce que des perçages de buses (5-5, 5-6) pour l'eau ou pour l'air soufflé débouchent dans le dessus de la partie inférieure (5) de la tête de pulvérisation et sont hermétiquement fermés par des parties de la partie supérieure (6) de la tête de pulvérisation qui sont situées au-dessus.

17. Appareil selon la revendication 15 ou 16, caractérisé par un capuchon de serrage (7) pouvant être enfilé sur les parties inférieure (5) et supérieure (6) de la tête de pulvérisation et comprenant de préférence des surfaces de guidage cunéiformes (7-1, 7-2, 7-3, 7-4) pour serrer les deux parties de la tête de pulvérisation.
